(19) Europäisches Patentamt
European Patent Office
Office européen des brevets

(11) Publication number: **0 498 268 A2**

(12) **EUROPEAN PATENT APPLICATION**

(21) Application number: **92101347.0**

(22) Date of filing: **28.01.92**

(51) Int. Cl.5: **C07K 5/06**, C07K 5/08, C07K 5/10, C07K 7/06, A61K 37/02

(30) Priority: **06.02.91 IT 30391**

(43) Date of publication of application: **12.08.92 Bulletin 92/33**

(84) Designated Contracting States: **DE ES FR**

(71) Applicant: **POLI INDUSTRIA CHIMICA S.p.A.**
**Piazza Agrippa, 1**
**I-20141 Milano(IT)**

(72) Inventor: **Poli, Stefano**
**Piazza Agrippa, 1**
**I-20141 Milano(IT)**
Inventor: **Coppi, Germano**
**Piazza Agrippa, 1**
**I-20141 Milano(IT)**

(74) Representative: **Minoja, Fabrizio**
**Studio Consulenza Brevettuale Via Rossini, 8**
**I-20122 Milano(IT)**

(54) **5-Oxo-L-proline derivatives and pharmaceutical use thereof.**

(57) 5-Oxo-L-proline ester and amide derivatives with amino acids and oligopeptides, the processes for the preparation thereof, pharmaceutical compositions containing them and the use thereof in therapy.

EP 0 498 268 A2

The present invention relates 5-oxo-L-proline derivatives of general formula (I)

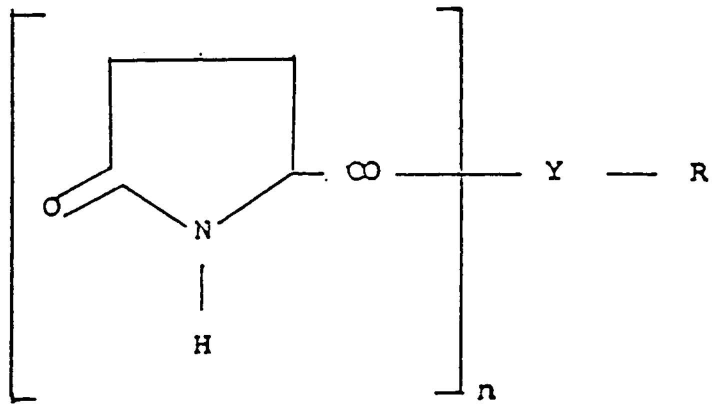

(I)

wherein

Y is O, S, NH or N*, where N* is a pyrrolidine or thiazolidine nitrogen atom;

R, when Y = O or S, is such that R - YH is a $C_2$-$C_5$ hydroxy- or thiol-alkylamine ; a $C_3$-$C_{11}$ L-hydroxy- or thiol-amino acid , which can be alifatic or aromatic; a hydroxy- or thiol-oligopeptide containing 2-6 amino acidic units, or an ester, amide or N-acylderivative thereof; or

R, when Y = NH or N*, is such that R - YH is a $C_4$-$C_{15}$ L-amino acid (or an ester, amide or N-acylderivative thereof), or a oligopeptide containing 2-6 amino acidic units (or an ester, amide or N-acylderivative thereof); or, when Y = NH, is $C_1$-$C_6$ alkyl, $C_6$-$C_{10}$ optionally substituted aryl, $C_7$-$C_{10}$ aralkyl;

n is the integers 1, 2 or 3, with the proviso that, when n > 1, R is a residue bearing at least two YH groups, which can be the same or different.

Preferred compounds according to the invention are those in which:

a) when Y = O, S, NH or N*, RYH is: 2-aminoethane(thi)ol, 2,3-diaminoethane(thi)ol; cysteamine, dopamine, tyramine, serine, threonine, tyrosine, 5-hydroxytryptophan, 2-mercaptohistidine, hydroxyproline, and related N-acylderivatives; biologically active hydroxybetaines; phosphoserine.

b) when Y = NH or N*, RYH is - besides the compounds cited in a) above - histamine, tryptamine, homocysteine thiolactone, creatine, essential amino acids, oligopeptides consisting of 2-6 essential amino acids.

By N-acylderivatives, N-acetyl, N-succinyl and N-benzoyl derivatives are particularly meant. $C_7$-$C_{10}$-aralkyl is preferably benzyl or phenylethyl. $C_6$-$C_{10}$-aryl is preferably phenyl optionally substituted by one or more halogen atoms, hydroxy, $C_1$-$C_6$ alkoxy, acetoxy, amino groups.

An oligopeptide containing 2-6 amino acids preferably comprises of L-thiazolidine-4-carboxylic acid, leucine, asparagine, valine, lysine, glycine, cysteine, phenylalanine, serine. Di- or tri-peptides containing said amino acids are particularly preferred.

The present invention also relates to the preparation of pharmaceutically acceptable salts of those compounds having groups which can be salified.

Italian Patent n. 1.202.426 and EP-A-90102388.7 disclose 3-N-(5-oxo-L-prolyl)-L-thiazolidine-4-carboxylic acid and derivatives thereof having immunostimulating, antitoxic, antioxidant, antiinflammotory and antiageing activities.

Now it has been found that L-pyroglutamic acid derivatives of formula (I) and the salts thereof with pharmaceutically acceptable acids and bases have improved pharmacological activities.

The compounds of the invention can be prepared according to conventional schemes generally used in the peptide synthesis as well as in the preparation of esters, thioesters or amides.

For example, 5-oxo-L-proline or the reactive derivatives thereof (acid chloride, anhydride, imidazolide) can be reacted with compounds of formula $R_1H$ (where $R_1$ has the same meanings as R or it can be converted into R by removing any protective groups). When using the acid chloride, the reaction will be preferably carried out in anhydrous solvents in the presence of acid binding agents. Alternatively, condensing agents such as dicyclohexylcarbodiimide or reactive esters can be used.

Compounds (I) have interesting pharmacological properties together with a very low acute toxicity. More specifically, the compounds of the invention have interesting immunostimulating, antiradical, antitoxic, antishock and antiageing properties.

The compounds of the invention have been tested in some immunologic tests in comparison with 3-L-pyroglutamyl-L-thiazolidine-4-carboxylic acid (PGT) (Italian Pat. n. 1.202.426).

The evidence of a stimulation of superoxide anion production in macrophages induced by the compounds of the invention is a very important test to evaluate the immunostimulating activity, since the production of superoxide anion ($O^{\cdot}_{2}$) plays an important role in microbicidal activity of macrophages (N.P. Cumming, M.J. Pabst, J.Exp. Med., 152, 1659, 1980). Therefore, $O^{\cdot}_{2}$ production in peritoneal macrophages isolated from prednisolone-immunodepressed animals treated with the test compounds was evaluated. Mice were treated during 11 days with 0.5 mg/kg/die/s.c. of prednisolone and with the test compounds at a dose of 100 mg/kg/i.p. two times a day, whereas the morning of the killing they received only the test compounds. After that, macrophages were isolated by means of peritoneal lavage and cultured in RPMI-1640 added with 10% foetal calf serum, at a concentration of $1x10^6$ cells/ml and at the temperature of +37°C. 1 ml aliquots of the cell suspension were incubated at 37°C for 15 min. with cytochrome C from horse hearth and stimulated with phorbol myristate acetate (H. Nielsen, Eur. J. Clin.Pharmacol., 30, 99, 1986). Then a spectrophotometric determination of reduced ferricytochrome C, using an extinction coefficient E(550) = 29.5 mM, was carried out at 550 nm. Treatment with prednisolone induces an immunosuppression which is evidenced by a lower production of superoxide anion; the simultaneous treatment with the compounds of the invention stimulates macrophages to produce $Or^{\cdot}_{2}$ up to values which are similar to those of not immunodepressed animals. Some compounds show a higher activity than that of PGT, others a comparable activity, at the same dosage.

The compounds were also subjected to the endoperitoneal infection test in the mouse. COBS CD-1 Male mice, weighing 22-25 g, were intraperitoneally inoculated with 0.5 ml/mouse of a suspension of pathogenic E. coli (in normal saline +0.5% pig gastric mucin), which shows a transmittance of about 50% at 600 nm. The mice are treated with the compounds two times a day, starting two days before, until the morning of the infection, 7 hours before the inoculation. Mortality is determined for about 7-8 days from the infection. The compounds of the present invention show a protective activity against the infection. Said activity, which is due obviously to a stimulation of the immune system, is higher or equal to that of the control drug PGT after oral and intraperitoneal administrations.

The compounds were found to be active also in the dinitrochlorobenzene-delayed hypersensitivity test in prednisolone-immunodepressed mice (A.C. Corsini and coll., J.Immunol.Method., 30, 195, 1979). Male COBS CD-I mice weighing about 30 g, are sensitized with 25 µl of dinitrochlorobenzene (DNCB) at the concentration of 20 mg/ml of acetone administered on an abdomen area which had been depilated at least two hours before the test. After 12 days, the challenge is carried out administering 5 µl of DNCB at the concentration of 10 mg/ml of acetone on the outer area of the auricle of the left ear, previous slight ether anaesthesia. The mice are treated with the test compounds from two days before to the sensitization day and with the compounds plus prednisolone (0.5 mg/kg/die s.c.; 0.05 ml/10 g) from the 10th day after sensitization to the challenge day. The values are expressed as the ratio of left ear weight (sensitized) to right ear weight (not sensitized). Treatment with the compounds of the present invention increases the delayed hypersensitivity reaction (IV type) in the prednisolone-immunodepressed animals at a higher or equal degree compared to the control drug (PGT) after oral and intraperitoneal administrations.

The compounds of formula (I) in which sulphur compounds are present (L-cysteine, 2-mercaptohistidine, etc.) also show an antiradical activity.

The antiradical activity was tested on doxorubicin mortality in the mouse (Olson R.D. et al. Life Sci., 29, 1393, 1981). Groups of 10 male mice CD-I (C. River), weighing 18-20 g, are treated orally with the test compounds; after 1 hour, doxorubicin is administered at a dose of 22 mg/kg i.p. (10 ml/kg). Treatment is repeated with the test compounds after 6 hours from doxorubicin administration and also the day after with the same frequency (2 times a day with a 7 hour interval between two treatments). Animal mortality is monitored for 14 days. The doxorubicin-treated group has a 80-90% mortality; the sulphur compounds of formula (I), at a dose of 0.5 mM/kg os 4 times in 2 days, show a protection against mortality of 40 and 50%, which is comparable to that of L-cysteine and N-acetylcysteine at the same molar doses.

The compounds of the present invention are active also on paracetamol intoxication in the rat at dosages comparable to those of PGT.

Compounds (I) are active as anti-shock drugs in the hypovolemic shock test in the rat (Coppi G., Falcone A., Europ. J. of Pharmacol. 182, 185, (1990)). Moreover, the compounds of the invention have a radio-protecting activity against ionizing radiations which is similar to the one of PGT (Italian Pat. N. 1.202.426, EP-A-90102388.7).

Moreover, the compounds of general formula (I) are active in improving neurocerebral performances in the elderly rat, in improving sexual performances in the male elderly rat, with a decrease in latencies and an increase in the frequency of sexual activity.

Finally, the compounds can be used to counteract excesses in oxidative processes, such as those deriving from chronic inflammatory conditions or from exposure to ionizing radiations.

Acute toxicities of compounds (I) by the oral and intraperitoneal routes are very low in the mouse and in the rat, analogously to PGT; they are higher than 2000 mg/kg.

What stated above clearly shows that the compounds of formula (I) can be used in therapy in a variety of pathological conditions, for example for the treatment of immunodeficiencies, auto-immune diseases, shock and ageing processes.

The present invention also relates to the various pharmaceutical compositions suitably formulated for the oral use, such as hard and soft gelatin capsules, sugar-coated pills, tablets, sachets, drops, syrups, sustained-release forms, and for the parenteral use, such as vials or solutions.

The pharmaceutical compositions of the invention are prepared with conventional techniques, using compatible pharmaceutically acceptable excipients and carriers, and they can also contain other active ingredients, having a complementary or anyhow useful activity. The unitary dose of compound (I) will generally range from 10 to 500 mg.

The following examples further illustrate the invention.

## EXAMPLE 1

N-(5-oxo-L-prolyl)-L-5-hydroxytryptophan (I, n = 1, Y = NH, RYH = L-5-HTP).

5 g (0.02 m) of L-5-hydroxy-tryptophan ethyl ester and 2.6 g (0.02 m) of 5-oxo-L-proline are dissolved in 30 ml of dimethylformamide (DMF). The reaction mixture is cooled to 0 ° C and 4.3 g (0.0205 m) of dicyclohexylcarbodiimide (DCC) are added thereto. After 24 hours at room temperature, the reaction mixture is filtered, solvent is evaporated off at 40 ° C and the residue is taken up with 50 ml of water. The precipitate is filtered, the solution is added with 0.8 g of NaOH dissolved in a few water and the mixture is left at room temperature for some hours, then it is treated with charcoal, filtered and passed through a column containing IR-120 in the $H^+$ form. The acid solution is evaporated to dryness at 30 ° C, the residue is taken up with a few acetone, insolubles are filtered off and the product is precipitated with ether, in form of a whitish powder. M.p. 134 ° C. The dicyclohexylamine salt is obtained in acetone in the form of white crystals. M.p. 121 ° C.

## EXAMPLE 2

N-Acetyl-5-(5-oxo-L-prolyl)-oxy-L-tryptophan (I, n = 1, Y = 0, RYH = N-Acetyl-L-5-HTP).

3,5 g (0.0133 m) of N-acetyl-5-hydroxy-L-tryptophan are dissolved in acetone and treated with 2.3 g of diphenyldiazomethane at 0-5 ° C. After complete decolourization, solvent is evaporated under vacuum, the residue is taken up in ethyl acetate, washed with sodium bicarbonate, dried and evaporated. The residue is taken up in ether to crystallize. 4.2 g are obtained.

1.7 g of this compound, 0.65 g of 5-oxo-L-proline and 10 mg of pyrrolidino-pyridine are dissolved in 10 ml of tetrahydrofuran and 3 ml of DMF, the solution is cooled to 0 ° C and added with 1.03 g of DCC. After 36 hours at room temperature the reaction mixture is filtered, solvent is evaporated off under vacuum and the residue is taken up in ethyl acetate, washed with sodium bicarbonate, and the solution is dried and evaporated. By trituration, the solid product is obtained. M.p. 171-2 ° C.

1 g of the above compound is hydrogenated with 0.1 g of 5% Pd/C in 10 ml DMF at room temperature. After filtration, DMF is evaporated off at 30 ° C/0.1 mm, the residue is taken up in THF, filtered with charcoal and evaporated again. The residual oil is triturated with ether/water to solidify, then the product is filtered and quickly dried under vacuum. M.p. 213-4 ° C.

## EXAMPLE 3

(5-oxo-L-prolyl)-L-tyrosyl-glycyl-glycine (I, n = 1, Y = NH, RYH = L-Tyr-Gly-Gly-OH).

A mixture of 3.81 g (0.0128 m) of p-GluTyrOH, 5.04 g (0.0128 m) of L-Glycylglycine benzyl ester tosylate and 1.73 g (0.0128 m) of N-hydroxybenzotriazole in 20 ml of DMF cooled to 0 ° C is added with 1.8 ml of triethylamine (0.0128 m) and 2.63 g (0.0128 m) of DCC. After stirring for 24 hours at room temperature, the reaction mixture is filtered and solvent is evaporated off under vacuum obtaining an oil which is partitioned between a sodium bicarbonate saturated solution and chloroform containing 5-10% methanol (3-4 times). The combined extracts are evaporated to constant weight obtaining a gummy residue which is taken up in 30 ml of chloroform containing 10% methanol and passed through a silica gel column

(30 mm d.), eluting with a chloroform/methanol gradient from 95:5 to 85:15. After a first fraction which contains impurities having a higher Rf, the product (4 g) is recovered, which is taken up in 60 ml of ethanol, added with 0.6 g of 5% Pd/C and hydrogenated at room temperature, then the catalyst is filtered off and solvent is evaporated off and the residue is taken up in 50 ml of abs. ethanol. After one night, the precipitate is filtered and dried at 60°/0.1 mm, obtaining 2.45 g of the title product (75.5%). M.p. 204-7°C.

## EXAMPLE 4

3-N-(5-oxo-L-prolyl)-L-thiazolidine-4-carbonyl-L-cysteine (I, n=1, Y=N*, RYH=L-Thz-L-Cys-OH).

6.82 g (0.02 m) of L-cystine dimethyl ester dihydrochloride in 20 ml of DMF are treated at 0-10°C with 5.6 ml (0.04 m) of triethylamine.

An equal volume of ethyl ether is added, the precipitate is filtered, ether is evaporated off and the solution is added to a suspension of 9.76 g (0.04 m) of (5-oxo-L-prolyl)-L-thiazolidine-4-carboxylic acid in 40 ml of DMF. The reaction mixture is stirred at 0°C until complete dissolution, then 8.24 g (0.04 m) of DCC are added. The reaction mixture is stirred for 6 hours at room temperature, then it is filtered and solvent is evaporated off under vacuum. The residue is taken up in water, the solution is filtered and evaporated to dryness and kept for some hours at 40°C/0.1 mm obtaining a friable foam. 13.6 g of this foam are treated with an excess of IR-120 ($H^+$), the solution is evaporated to dryness, the residue is stripped twice with methanol and dried for 12 hours at 45°C/0.1 mm, obtaining a product (10 g. 69.4%) consisting of N,N'-bis[-(5-oxo-L-prolyl)-L-thiazolidine-4-carbonyl]-L-cystine dimethyl ester.

14.4 g of this compound (0.02 m) in 80 ml $H_2O$ are dropped in 45' into 40 ml of 1N NaOH at room temperature. After 15', the mixture is treated with 40 ml of IR-120 ($H^+$), stirred for 15', then filtered. The solution is concentrated to 50 ml, then it is diluted with 100 ml of methanol. The mixture is left to crystallize, filtered and dried at 80°C, obtaining 7.38 g of N,N'-bis[(5-oxo-L-prolyl)-L-thiazolidine-4-carbonyl]-L-cystine. M.p.180-4°C.

6.92 g of this compound (0.01 m) in 50 ml of 50% acetic acid at 40°C are added with 5 g of zinc powder and the suspension is kept for 1.5 hours at 50°C, then it is filtered, the solution is treated with hydrogen sulfide, filtered again and evaporated to dryness to obtain 6.7 g of a foam consisting of a product unitary by TLC (Rf =0.3; $CHCl_3$ 50/MeOH 15/$NH_4OH$ 2).

## EXAMPLE 5

3-N-(5-oxo-L-prolyl)-L-thiazolidine-4-carbonyl-L-phenylalanyl-L-lisinamide hydrochloride (I, n=1, Y=N*, RYH=L-Thz-L-Phe-L-Lys-$NH_2$.HCl).

6.5 g (0.03 m) of L-phenylalanine methyl ester hydrochloride in 60 ml of methylene chloride are added with 4.2 ml (0.03 m) of triethylamine.

After 10' the reaction mixture is cooled to 0°C and 7.32 g (0.03 m) of 3-N-(5-oxo-L-prolyl)-L-thiazolidine-4-carboxylic acid are added, followed by 6.18 g (0.03 m) of DCC. After 5 hours the reaction mixture is filtered, evaporated, the residue is taken up in ethyl acetate and the precipitate is filtered. The solution is concentrated and left to crystallyze. The filtered product is triturated in water to obtain (5-oxo-L-prolyl)-L-thiazolidine-4-carbonyl-L-phenylalanine methyl ester. M.p. 149-51°C.

5.28 g of this compound are stirred for 15' with 80 ml of 1N NaOH. The solution is filtered and acidified and the precipitated product is filtered, obtaining 4.5 g of the acid. M.p. 206-8°C ($H_2O$).

5.08 g of this compound (0.013 m) in 25 ml of DMF at 0°C are added with 3.18 g (0.013 m) of H-Lys-(BOC)$NH_2$ (0.013 m), 1.76 g (0.013 m) of N-hydroxybenzotriazole and 2.7 g (0.0131 m) of DCC.

After 48 hours at room temperature, the reaction mixture is filtered and solvent is evaporated off. The residue is taken up in 50 ml of sodium bicarbonate and 70 ml of methylene chloride and it is left to stand for 12 hours at 0°C.

The solid is filtered and dried, obtaining 7.1 g. M.p. 125-130°C. This compound (3.22 g) is suspended in 65 ml of dioxane, 10 ml of 15% HCl in dioxane are added and the mixture is stirred until the jelly mass is transformed into a crystalline suspension which is quickly filtered, washed with dioxane, ether, and dried in a dryer over KOH. 3.75 g of a product unitary in TLC are obtained (Rf = 0.4 in butanol 50/pyridine 12/acetic acid 12/$H_2O$ 25).

## EXAMPLE 6

3-N-(5-oxo-L-prolyl)-L-thiazolidine-4-carbonyl-L-lysyl-L-leucyl-L-lysinamide dihydrochloride (I, n = 1, Y = N*, RYH = L-Lys-L-Leu-L-Lys-$NH_2$.2HCl).

10.1 g (0.018 m) of Z-Lys(BOC)OH.DCHA, 3.27 g (0.018 m) of $LeuOCH_3$ and 2.43 g (0.018 m) of N-hydroxybenzotriazole are suspended in 50 ml of DMF at 0°C and they are added with 3.71 g (0.018 m) of DCC in 10 ml of DMF. After 24 hours, the reaction mixture is filtered and solvent is evaporated off, the residue is taken up in ethyl acetate, the organic phase is washed with sodium bicarbonate, dried, concentrated and diluted with petroleum ether to obtain a precipitate of Z-Lys(BOC)-$LeuOCH_3$.M.p. 109-10°C (7.7 g, 84.4%).

5.65 g of this compound (0.011 m) in 56 ml of methanol/dioxane 1/1 are added with 15 ml of 1N NaOH and the reaction mixture is left for 15' at room temperature. Solvent is evaporated off under vacuum and the residue is taken up with a few water. The mixture is acidified with 15 ml of N HCl and extracted with ethyl acetate. This compound is dried, concentrated to small volume and diluted with petroleum ether which causes Z-Lys(BOC)-LeuOH to crystallize. M.p. 80-3°C.

4.5 g (0.009 m) of this compound in 20 ml of DMF are added with 2.24 g (0.009 m) of H-Lys(BOC)$NH_2$, 1.23 g (0.009 m) of N-hydroxybenzotriazole and 1.9 g (0.0092 m) of DCC. After 15 hours at room temperature, the reaction mixture is filtered, solvent is evaporated off, the residue is taken up in aqueous sodium bicarbonate/methylene chloride, the methylene phase is separated which, by evaporation and drying at 60°C/0.1 mm, gives 6.4 g of Z-Lys(BOC)-Leu-Lys(BOC)$NH_2$. M.p. 170-2°C. 6.5 g (0.009 m) of this compound are dissolved in 100 ml of methanol and hydrogenated at room temperature with 2 g of 5% Pd/C. After filtration, solvent is evaporated off, the residue is taken up in ethyl acetate, then diluted with petroleum ether, obtaining 3.46 g of H-Lys(BOC)-Leu(BOC)$NH_2$. M.p. 106-9°C. This compound (0.00585 m) in 15 ml of DMF is added with 1.42 g (0.0058 m) of 3-N-(5-oxo-L-prolyl)-L-thiazolidine-4-carboxylic acid, 0.79 g (0.0058 m) of N-hydroxybenzotriazole and 1.22 g of DCC at 0°C. After 15 hour stirring at room temperature, the mixture is filtered, solvent is evaporated off and the residue is partitioned between aqueous sodium bicarbonate and methylene chloride. The solid is filtered and dried at 40°C/O, 1 mm, obtaining 4.45 g of pGlu-Tzh-Lys(BOC)-Leu-Lys(BOC)$NH_2$. 4.14 g of this compound in 25 ml of acetic acid are added with 40 ml of 4% HCl in aqueous acetic acid, in 3 portions, during 30'. An oil is separated, is decanted and repeatedly washed with ethyl ether. The product is then quickly filtered and dried at 40°C/0.1 mm, obtaining 2.9 g of product. M.p. 245-7°C.

**EXAMPLE 7**

N,N'-bis(5-oxo-L-prolyl)-L-lysine (I, n = 2, Y = NH, NH, RYH = Lys).

9.05 g (0.05 m) of dicyclohexylamine are dropped during 15' into a stirred solution of 14.5 g (0.025 m) of L-lysine benzyl ester paratoluenesulfonate at 0°C and 14.75 g (0.05 m) of pentafluorophenyl-L-pyroglutamate in 50 ml of DMF. After warming to room temperature, the mixture is left to stand overnight, then it is diluted with 200 ml of ethyl ether and dicyclohexylamine tosylate is filtered. The solution is evaporated first at 15 mm, then at 0.1 mm, the resinous residue is taken up in 100 ml of water and the solution is treated for 30' in 100 ml of IR-120 ($H^+$). The resulting mixture is filtered and the solution is treated with 100 ml of IRA-93, filtered and concentrated to dryness. The residue is taken up into boiling ethanol, cooled to 0-5°C and it is left one night at this temperature. 2.5 g of bis-(5-oxo-L-prolyl)-L-lysine benzyl ester are obtained. M.p. 153-5°.

This compound is dissolved in 80% ethanol, hydrogenated at room temperature in the presence of 1 g of 5% Pd/C. The catalyst is filtered and the mixture is evaporated to dryness, the foamy residue is taken up in 50 ml of ethanol and boiled. Upon cooling, the product separates in amorphous form, unitary in chromatography (Rf = 0.4 in butanol 4/acetic acid l/$H_2O$ l).

By means of similar procedures, the compounds reported in Table 1 below are prepared.

Table 1

| n | Y | R'YH | M.p. or RF |
|---|---|---|---|
| 1 | N* | L-Tzh-L-Val $OCH_3$ | 154-6°C |
| 1 | N* | L-Tzh-L-Leu $OCH_3$ | 133.5-134.5°C |
| 1 | N* | L-Tzh-L-Asn $OCH_3$ | 0.6 (1) |
| 1 | N* | L-Tzh-L-Leu OH | 0.45 (2) |
| 1 | N* | L-Tzh-L-Val OH | 0.40 (2) |
| 1 | N* | L-Tzh-L-Lys $OCH_3$ | 0.40 (3) |
| 1 | N* | L-Tzh-Gly OH | 0.60 (3) |
| 1 | N* | L-Tzh-L-Phe OH | 208-10°C |
| 1 | N* | L-Tzh-L-Phe-L-Lys OH | 0.45 (2) |
| 1 | N* | L-Tzh-L-Lys-L-Phe OH | 0.4 (2) |
| 1 | NH | L-Phe-Gly-OH | 0.6 (2) |
| 1 | (N*), NH | L-Phe-L-Ser OH | 0.65 (2) |
| 2 | NH, O | L-Ser OH | 0.4 (2) |
| 2 | NH, O | L-5-HTP | 224-6°C |
| 2 | NH, O | L-Thr OH | 0.45 (2) |
| 2 | NH, O | L-Tyr | 0.5 (3) |
| 1 | S | 2-HS-L-Hys | 0.6 |
| 2 | N*, O | L-Hyp | 0.45 (2) |
| 2 | NH, NH | L-Arg | 0.25 |
| 1 | NH | L-Hys | 0.3 |

(1) $CHCL_3$ 30/acetone 20/MeOH 10

(2) n-BuOH 4/AcOH 1/$H_2O$ 1

(3) $CHCl_3$ 30/MeOH 15 / AcOH 5

N* = proline or thiazolidine nitrogen

L-Tzh = L-thiazolidine-4-carboxylic acid

5-HTP = 5-hydroxytryptophan

2-HS-Hys = 2-mercapto histidine

Hyp = hydroxyproline

## Claims

### Claims for the following Contracting States: DE, FR

1. Compounds of formula (I)

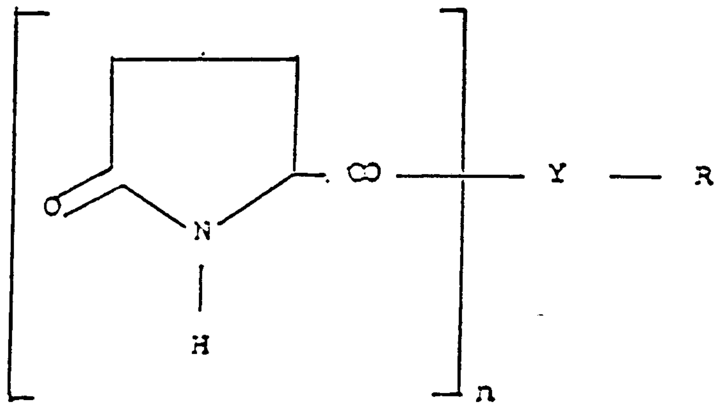

(I)

wherein
Y is O, S, NH or N*, where N* is a pyrrolidine or thiazolidine nitrogen atom;
R, when Y = O or S, is such that R - YH is a $C_2$-$C_5$ hydroxy- or thiol-alkylamine ; a $C_3$-$C_{11}$ L-hydroxy- or thiol-amino acid , which can be alifatic or aromatic; a hydroxy- or thiol-oligopeptide containing 2-6 amino acidic units, or an ester, amide or N-acylderivative thereof; or
R, when Y = NH or N*, is such that R - YH is a $C_4$-$C_{15}$ L-amino acid (or an ester, amide or N-acylderivative thereof), or a oligopeptide containing 2-6 amino acidic units (or an ester, amide or N-acylderivative thereof);
or, when Y = NH, is $C_1$-$C_6$ alkyl, $C_6$-$C_{10}$ optionally substituted aryl, $C_7$-$C_{10}$ aralkyl;
n is the integers 1, 2 or 3, with the proviso that, when n > 1, R is a residue bearing at least two YH groups, which can be the same or different.

2. Compounds according to claim 1 in which, when Y = O, S or NH or N*, RYH is: 2-aminoethane(thi)ol, 2,3-diaminoethane(thi)ol;cysteamine, dopamine, tyramine, serine, threonine, tyrosine, 5-hydroxytryptophan, 2-mercaptohistidine, hydroxyproline, and related N-acylderivatives; biologically active hydroxybetaines; phosphoserine.

3. Compounds according to claim 1 in which, when Y = NH or N*, RYH is - besides the compounds cited in claim 2 - histamine, tryptamine, homocysteine thiolactone, creatine, essential amino acids, oligopeptides consisting of 2-6 essential amino acids.

4. Pharmaceutical compositions containing as the active ingredient one compound of claims 1-3 in admixture with a pharmaceutically acceptable carrier.

5. A compound of claims 1-3 for the use as a therapeutical agent.

6. The use of a compound of claims 1-3, or of a salt thereof, for the preparation of a medicament for the treatment of diseases of the immune system, auto-immuno diseases, ageing processes and shock.

**Claim for the following Contracting State : ES**

1. A process for the preparation of the compounds of formula (I)

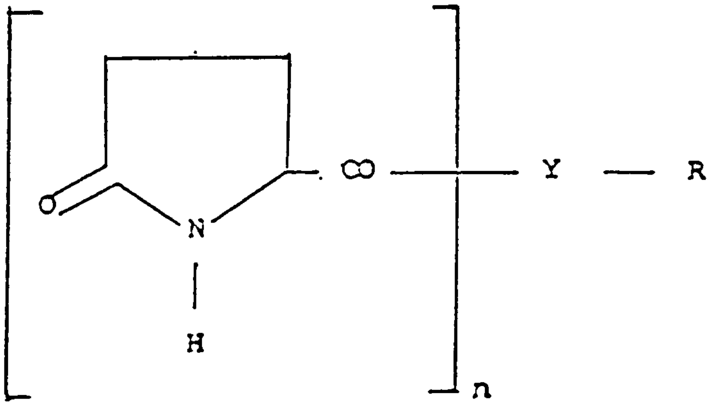

(I)

wherein

Y is O, S, NH or N*, where N* is a pyrrolidine or thiazolidine nitrogen atom;

R, when Y = O or S, is such that R - YH is a $C_2$-$C_5$ hydroxy- or thiol-alkylamine ; a $C_3$-$C_{11}$ L-hydroxy- or thiol-amino acid , which can be alifatic or aromatic; a hydroxy- or thiol-oligopeptide containing 2-6 amino acidic units, or an ester, amide or N-acylderivative thereof; or

R, when Y = NH or N*, is such that R - YH is a $C_4$-$C_{15}$ L-amino acid (or an ester, amide or N-acylderivative thereof), or a oligopeptide containing 2-6 amino acidic units (or an ester, amide or N-acylderivative thereof);

or, when Y = NH, is $C_1$-$C_6$ alkyl, $C_6$-$C_{10}$ optionally substituted aryl, $C_7$-$C_{10}$ aralkyl;

n is the integers 1, 2 or 3, with the proviso that, when n > 1, R is a residue bearing at least two YH groups, which can be the same or different; in which process 5-oxo-L-proline, or a reactive derivative thereof, is reacted with a compound of formula

$R_1H$

wherein $R_1$ has the same meanings as R, or it can be converted into R by removing any protective groups present.